Europäisches Patentamt

European Patent Office

Office européen des brevets

(19)

(11) EP 0 572 341 B1

(12) FASCICULE DE BREVET EUROPEEN

(45) Date de publication et mention
de la délivrance du brevet:
**14.10.1998 Bulletin 1998/42**

(51) Int Cl.⁶: **G01N 33/02**

(21) Numéro de dépôt: **93430006.2**

(22) Date de dépôt: **23.04.1993**

(54) **Procédé et unité pour analyser automatiquement les paramètres physico-chimiques sur un échantillonnage de fruits**

Verfahren und Einheit zur automatischen Analyse der physikalisch-chemischen Parameter einer Stichprobe von Früchten

Method and apparatus for analysing automatically the physico-chemical parameters of a sample of fruits

(84) Etats contractants désignés:
**CH DE ES GB IT LI NL**

(30) Priorité: **29.05.1992 FR 9206802**

(43) Date de publication de la demande:
**01.12.1993 Bulletin 1993/48**

(73) Titulaire: **SOCIETE D'EXPLOITATION DU SYSTEME TOP - SETOP**
**F-84300 Cavaillon (FR)**

(72) Inventeur: **Giraud, Charles**
**F-84460 Cheval Blanc (FR)**

(74) Mandataire: **Somnier, Jean-Louis et al**
**c/o Cabinet Beau de Loménie,**
**232, Avenue du Prado**
**13295 Marseille Cédex 08 (FR)**

(56) Documents cités:
EP-A- 0 278 636          WO-A-88/07710
FR-A- 2 528 332          US-A- 3 773 172

## Description

La présente invention a pour objet un procédé pour analyser automatiquement les paramètres physico-chimiques sur un échantillonnage de fruits ou similaire, et une unité pour la mise en oeuvre dudit procédé.

Le secteur technique de l'invention est celui des procédés et des dispositifs comprenant des appareils de laboratoire pour l'analyse des qualités organoleptiques des produits destinés à la consommation humaine.

De telles analyses sont quelquefois effectuées dans les stations de conditionnement de façon manuelle et empirique. On connaît aussi une machine pour tester les fruits en vue de mesurer leur teneur en sucre. Une telle machine est décrite dans le brevet FR. 2 528 332.

Le brevet EP-A-0 278 636 décrit un procédé d'analyse de fourrage, d'herbage, de fruits ou de légumes pour en déterminer la valeur nutritionnelle ; le procédé peut être mis en oeuvre par une unité portable comportant un dispositif à micro-processeur.

L'objectif de la présente invention est de déterminer sur un lot d'échantillonnage les particularités organoleptiques des fruits ou similaire, en vue d'en apprécier : la qualité gustative, les possibilités de conservation, l'aptitude à la transformation industrielle.

Cet objectif est atteint par le procédé selon l'invention pour analyser automatiquement les paramètres physico-chimiques sur un échantillonnage de fruits, au moyen d'une unité robotisée comprenant un automate programmable que l'on relie à un ordinateur, se caractérisant par les étapes suivantes :

a) on réceptionne sur des moyens récepteurs à défilement pas à pas les fruits d'un lot d'échantillonnage ;
b) on pèse chaque fruit dudit échantillonnage et on entre les mesures pondérales dans la mémoire dudit ordinateur ;
c) on mesure la dureté desdits fruits et on entre lesdites mesures dans la mémoire dudit ordinateur ;
d) on extrait le jus desdits fruits de l'échantillonnage ;
e) on mesure le taux de sucre contenu dans lesdits fruits au moyen d'un réfractomètre électronique et on entre lesdites mesures dans la mémoire dudit ordinateur ;
f) on collecte la totalité du jus extrait précédemment ;
g) on pèse la totalité du jus extrait et on entre cette mesure dans la mémoire dudit ordinateur ;
h) on collecte parallèlement à l'étape f) une partie dudit jus extrait ;
i) on mesure l'acidité de ladite partie du jus extrait du lot d'échantillonnage par réaction acido-basique et on entre cette mesure dans la mémoire dudit ordinateur ;

lequel ordinateur donne les résultats de l'analyse et les qualités organoleptiques des fruits dont :

- le rapport $\dfrac{\text{poids total des fruits}}{\text{poids total du jus extrait,}}$
- la dureté individuelle et moyenne des fruits ainsi que les écarts types,
- le taux individuel et moyen de sucre ainsi que les écarts types,
- l'acidité moyenne des fruits.

L'objectif est également atteint par l'unité robotisée selon l'invention, pour l'analyse des paramètres physico-chimiques d'un échantillonnage de fruits, comprenant un automate programmable relié à un ordinateur et des moyens de commande des énergies nécessaires au fonctionnement de ladite unité, caractérisée en ce qu'elle comporte en combinaison :

a) des moyens récepteurs de défilement pas à pas des fruits d'un lot d'échantillonnage ;
b) des moyens pour peser chaque fruit dudit échantillonnage ;
c) des moyens pour mesurer la dureté desdits fruits ;
d) des moyens pour extraire le jus desdits fruits ;
e) des moyens pour mesurer le taux de sucre contenu dans le jus extrait ;
f) des moyens pour collecter la totalité du jus extrait ;
g) des moyens pour peser la totalité du jus extrait ;
h) des moyens pour collecter une partie dudit jus extrait ;
i) des moyens pour mesurer l'acidité de ladite partie du jus extrait du lot d'échantillonnage et en ce que les mesures effectuées en b), c), e), g) et i) sont entrées dans la mémoire dudit ordinateur, lequel compare lesdites mesures et les paramètres entrés dans sa mémoire et donne les résultats de l'analyse et les qualités

organoleptiques des fruits :

- le rapport $\dfrac{\text{poids total des fruits}}{\text{poids total du jus extrait,}}$
- la dureté moyenne des fruits,
- le taux moyen de sucre,
- l'acidité moyenne des fruits.

Ladite unité comporte un bâti et des moyens de commande des énergies nécessaires à son fonctionnement, et comporte en outre un plateau sensiblement horizontal, monté à rotation sur ledit bâti et comportant des orifices circulaires régulièrement répartis sur le périmètre du plateau, lequel est entraîné séquentiellement par un moto-réducteur, par fraction de tour correspondant à l'arc qui sépare deux orifices, lesquels sont d'un diamètre inférieur à celui des fruits, de telle sorte que les fruits placés sur ledit plateau, demeurent partiellement engagés dans lesdits orifices en attente de leur analyse.

Dans un mode de réalisation préférentielle, ledit plateau adopte une forme annulaire et est monté tournant sur des galets montés à rotation libre, sur ledit bâti, lequel plateau est soumis à l'action de galets de centrage en appui sur son bord périphérique et son entraînement en rotation est réalisé par un galet de friction soumis à l'action de moyens élastiques et calé sur l'arbre de sortie dudit moto-réducteur séquentiel.

Ledit plateau comporte un détecteur de présence des orifices, associé à un doigt d'arrêt escamotable situé sur le trajet desdits orifices, lequel doigt est d'une section inférieure à celle desdits trous, pour y pénétrer à l'intérieur et stopper séquentiellement le plateau dans une position précise.

Lesdits moyens pour peser chaque fruit se composent d'un détecteur de présence des fruits, associé à une coupelle escamotable d'un diamètre inférieur à celui des orifices du plateau, et situé sur le trajet desdits orifices, laquelle coupelle est portée par un capteur de force relié audit ordinateur et est soumise à l'action de moyens pour la faire pénétrer dans lesdits orifices, afin de soulever séquentiellement et en synchronisme avec le fonctionnement du plateau un fruit de l'échantillonnage en vue de sa pesée.

Lesdits moyens pour mesurer la dureté des fruits, se composent de moyens pour immobiliser chaque fruit séquentiellement en synchronisme avec les autres organes de l'unité, et d'un pénétromètre accouplé à un capteur de force, relié audit ordinateur, dont le poinçon est actionné en synchronisme pour pénétrer dans le fruit.

Dans un mode de réalisation préférentielle, lesdits moyens pour mesurer la dureté des fruits se composent d'une première coupelle fixe solidaire du bâti, située au-dessus du plateau et sur le trajet des orifices, au droit de laquelle s'immobilisent successivement les orifices du plateau et une seconde coupelle escamotable, coaxiale à ladite première coupelle, dont les concavités desdites coupelles sont en vis à vis ; ladite seconde coupelle est d'un diamètre inférieur à celui desdits orifices et est actionnée par des moyens télescopiques situés au-dessous dudit plateau, pour lui faire traverser tour à tour lesdits orifices et amener le fruit, quelle que soit sa grosseur, en appui sur ladite première coupelle, laquelle comporte un trou central à travers lequel passe le poinçon du pénétromètre.

Ladite unité comporte au droit de l'axe du pénétromètre un éjecteur des fruits ayant passé le poste de mesure de dureté, lequel éjecteur est parallèle audit plateau et est situé entre ledit plateau et ladite première coupelle fixe du pénétromètre.

Dans un mode de réalisation, lesdits moyens télescopiques de la seconde coupelle se composent de deux vérins superposés, dont l'un a pour fonction de déplacer ladite coupelle pour mettre le fruit en appui sur ladite première coupelle fixe, et dont l'autre vérin a pour fonction d'immobiliser momentanément la coupelle au-dessus dudit plateau, pour mettre le fruit sensiblement dans le champ d'action dudit éjecteur et transférer le fruit :

dudit plateau jusqu'audit moyen d'extraction du jus.

Avantageusement, l'extrémité du poinçon du pénétromètre comporte une concavité délimitée par un bord périphérique affûté, pour trancher la peau du fruit préalablement à la pénétration du poinçon dans la chair du fruit.

Lesdits moyens pour extraire le jus des fruits, sont constitués par un pressoir, disposé sensiblement horizontalement et au-dessous dudit plateau, lequel pressoir comporte un orifice latéral pour recevoir les fruits éjectés du plateau.

Dans un mode d'exécution, ledit pressoir se compose d'un cylindre ouvert à ses extrémités, dont la paroi comporte une ouverture pour l'introduction des fruits par gravité, dans lequel cylindre est monté un piston fixé à la tige mobile d'un vérin à double effet dont le corps est fixé au bâti pour déplacer le piston sur la longueur dudit cylindre, lequel cylindre est attelé à la tige mobile d'un vérin à double effet, dont le corps est fixé au bâti et dont la tige a pour fonction d'appuyer le cylindre contre une enclume fixée audit bâti, et pour l'éloigner de celle-ci, afin de laisser tomber par gravité la matière sèche du fruit après extraction du jus, sous la pression exercée par ledit piston sur le fruit en appui sur l'enclume, à laquelle enclume est fixée une tubulure d'écoulement du jus.

Le jus issu de ladite enclume est canalisé par une première tubulure pour passer à travers un réfractomètre en vue de mesurer le taux de sucre, et ensuite amener le jus dans un bac de réception dit de collecte totale. L'unité comporte en outre en amont dudit réfractomètre un té, dont le tube principal raccordé à ladite première tubulure est incliné en aval vers le réfractomètre et dont le tube de dérivation est situé au-dessous dudit tube principal et raccordé à une seconde tubulure aboutissant à un second bac de réception dit de collecte partielle, laquelle seconde tubulure comporte une première vanne située du côté dudit té pour recueillir, en position fermée de la vanne, un échantillon du jus de chaque fruit du lot et une seconde vanne située en aval de ladite première vanne pour recueillir en position fermée de ladite seconde vanne la totalité des échantillons du jus des fruits d'un lot d'échantillonnage.

Les bacs de réception de récolte totale et de récolte partielle sont chacun portés par un capteur de force fixé audit bâti et relié audit ordinateur.

Ladite unité comporte en outre une tubulure issue de la réserve d'une base et aboutissant dans ledit second bac de réception de collecte partielle, sur laquelle tubulure est insérée une vanne reliée à des moyens de commande pour délivrer dans ledit bac des doses de ladite base, un agitateur et une sonde de pH reliée audit ordinateur.

Ladite vanne de dosage de la base, ledit agitateur et ladite sonde sont montés sur un bras déplaçable verticalement et actionné par un vérin à double effet, pour plonger la sonde et l'agitateur dans ledit bac de récolte partielle ou pour les extraire dudit bac, afin de permettre le retrait des bacs de récolte totale et de récolte partielle en vue de leur nettoyage périodique.

Le résultat de l'invention est la détermination des particularités organoleptiques des fruits, par l'application dudit procédé mis en oeuvre par l'unité robotisée susdite.

L'appareillage proposé permet de généraliser les analyses des paramètres physico-chimiques sur un lot d'échantillonnage, de façon rigoureuse et économique. L'ensemble des opérations est pilotée par ordinateur, lequel assure également tous les calculs et l'analyse des résultats.

D'autres avantages et les caractéristiques de l'invention ressortiront encore à la lecture de la description suivante donnée à titre d'exemple non limitatif et en référence aux dessins annexes, sur lesquels :

- la figure 1 est une vue en perspective schématique d'une unité selon l'invention,
- la figure 2 est une vue en perspective schématique partielle de la table annulaire tournante et des moyens de mesure de la dureté des fruits, des moyens presseurs et de la prise d'échantillonnage du jus,
- la figure 2a est une vue en coupe de l'extrémité du poinçon du pénétromètre,
- la figure 3 est une vue en perspective schématique partielle de la table annulaire et de ses moyens d'arrêt et de pesage des fruits,
- la figure 4 est une vue en perspective schématique partielle de la partie de l'unité qui mesure automatiquement l'acidité et qui comporte les bacs de réception de collecte partielle et de collecte totale,
- la figure 5 est un diagramme de fonctionnement de ladite unité.

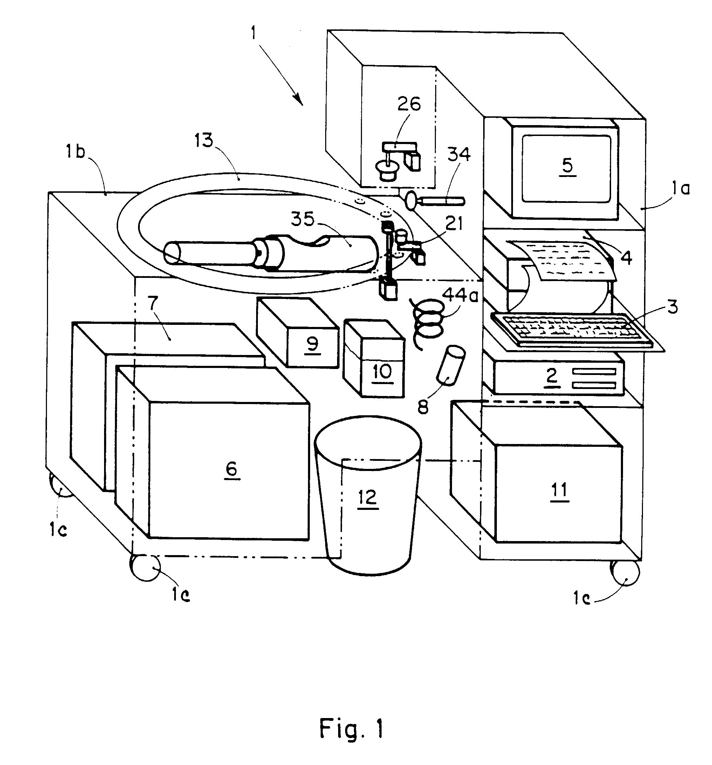

L'unité robotisée, telle que schématisée à la figure 1, se compose d'un bâti 1 monté sur des galets lc, comprenant une armoire informatique la dans laquelle sont placés un ordinateur 2, au-dessus de celui-ci un clavier 3 et une imprimante 4, et au-dessus de l'imprimante, un écran 5. Attenante à cette armoire se trouve une structure 1b pouvant former une enceinte et dans laquelle sont montés les appareillages d'analyse des fruits et les moyens de puissance de l'unité. A l'extrémité opposée à l'armoire 1a et à la partie basse avant, se trouvent un automate programmable et un module de commandes électriques 6. Au même niveau et à l'arrière, un module de commandes pneumatiques 7. Dans la partie située entre lesdits modules 6/7 et l'armoire la, sont montés un réfractomètre électronique 8 et son module électronique 9, laquelle armoire comprend également l'électronique des différents capteurs de force que comporte l'unité robotisée et qui seront décrits plus loin. Entre le réfractomètre 8 et son module 9, est disposé un réservoir de soude 10 relié à un module "acidité" 11, situé à la partie inférieure de l'armoire la, au-dessous de l'ordinateur 2. Ce module sera décrit en détail plus loin. Au-dessous du réservoir de soude 10, est placée une poubelle 12 dans laquelle tombent, par gravité, les déchets secs des fruits préalablement pressés pour en extraire le jus ainsi que les effluents de lavage.

Au-dessus de la partie 1b du bâti, est monté à rotation un plateau annulaire sensiblement horizontal 13, comprenant des moyens récepteurs pour y disposer les échantillons d'un lot de fruits, par exemple compris entre vingt et trente fruits.

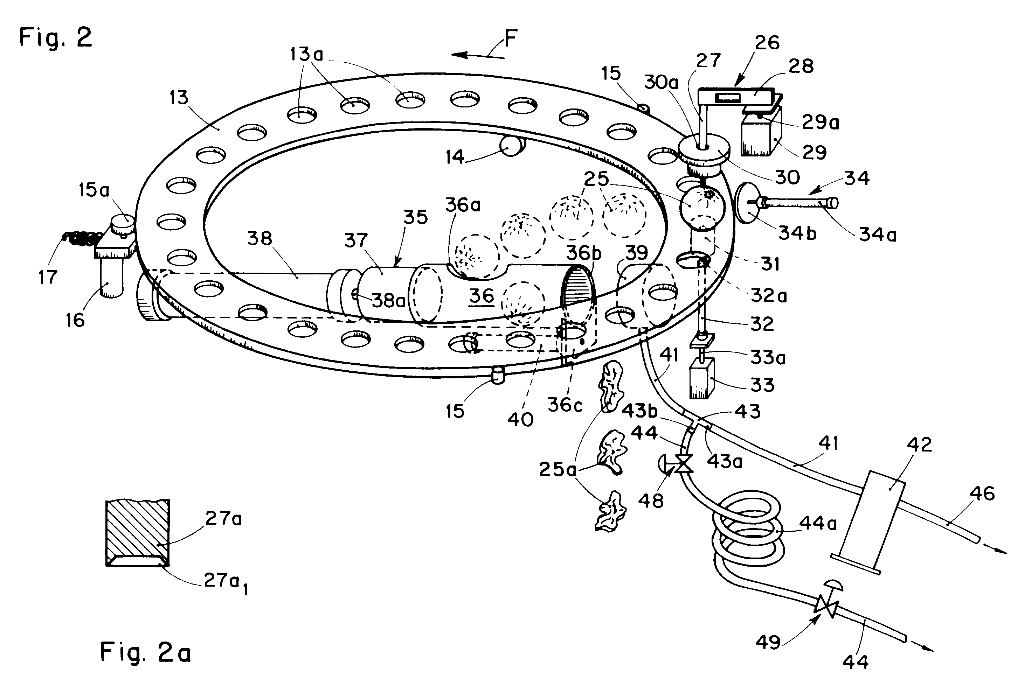

On se reporte maintenant aux figures 2 à 4 qui représentent avec plus de détail les différents organes qui composent l'appareillage de l'unité robotisée. Les fruits sont disposés manuellement sur ledit plateau annulaire 13, lequel comporte lesdits moyens récepteurs constitués par des trous 13a régulièrement répartis selon un pas. Ces trous 13a sont d'un diamètre légèrement inférieur à celui des plus petits fruits à analyser, par exemple leur diamètre est égal à cinquante millimètres. Ledit plateau 13 est monté tournant sur des galets porteurs 14, par exemple au nombre de trois, et est maintenu en place par des galets centreurs 15, par exemple au nombre de trois, dont l'un 15a est calé sur l'axe de sortie d'un moto-réducteur 16 poussé par un ressort 17 du côté du plateau et qui assure l'entraînement en rotation lente de celui-ci. Pour synchroniser la rotation et l'arrêt du plateau 13 avec les autres séquences du processus, le plateau comporte un dispositif de détection et de blocage dans une position précise. Ces moyens de détection et de blocage à position fixe, sont par exemple composés d'un détecteur optique de type réflex 18, situé sur le trajet des trous 13a et au-dessus du plateau 13 dont le réflecteur 18a est fixé à l'extrémité d'un verrou de blocage 19, situé coaxialement audit détecteur et au-dessous dudit plateau. Ledit verrou 19 est fixé à l'extrémité de la tige mobile 20a d'un vérin à double effet 20 qui a pour fonction d'effacer le verrou lorsque le plateau tourne d'une fraction de tour, soit d'un pas, et de le faire pénétrer dans un trou 13a pour immobiliser le plateau dans une position précise. Ce verrou 19 est d'un diamètre inférieur à celui des trous 13a et fonctionne en synchronisme avec ledit détecteur 18. L'alimentation électrique du moto-réducteur 16 est coupée en synchronisme avec ledit détecteur 18 et avec un léger retard, de telle sorte que le bord intérieur du trou 13a vient en fin de course au contact du verrou 19.

La pesée des fruits placés sur ledit plateau 13 est réalisée par un capteur de force 21 sur lequel est montée, à une extrémité, une coupelle 22. Le capteur de force 21 est fixé à son autre extrémité à la tige mobile 23a d'un vérin à double effet 23. Cet ensemble est situé au-dessous du plateau 13, de telle sorte que le dispositif de détection/blocage 18/19 est en aval desdits moyens de pesée 21/22 par rapport au sens de rotation F. La coupelle 22 est située sur le trajet des trous 13a. Au-dessus du plateau 13 et coaxialement à ladite coupelle 22, est monté un capteur de détection de présence des fruits 24, par exemple un détecteur optique de type réflex, dont le réflecteur 24a est fixé dans le fond de la coupelle 22.

Lorsque le capteur 24 détecte un fruit, le verrou de blocage 19 arrête le plateau 13 à position fixe. Le vérin 23 est actionné et la coupelle 22 venant au contact du fruit le soulève. La coupelle 22 est d'un diamètre inférieur à celui des trous 13a du plateau, pour passer à travers lesdits trous pendant cette opération. Le fruit étant séparé du plateau est alors pesé. Après stabilisation du signal de sortie du capteur de force 21, la mesure de poids est entrée dans la mémoire dudit ordinateur 2. Le vérin redescend, le fruit est de nouveau replacé dans le trou 13a du plateau. Le verrou de blocage 19 est ensuite escamoté au-dessous du plateau 13 et le motoréducteur 16 est excité pour faire tourner ledit plateau d'une fraction de tour, pour mettre le fruit suivant dans le champ du détecteur de présence 24/24a.

Les mesures de poids de chaque fruit sont mémorisées puis leur somme est calculée en fin de lot. Les détecteurs 18/24 peuvent bien entendu être de tout type connu, tel que optique, capacitif ou autre.

La mesure de dureté des fruits est réalisée à l'étape suivante en synchronisme avec la pesée du fruit du poste précédent. La dureté des fruits 25 est mesurée au moyen d'un pénétromètre 26 situé sur le trajet des trous 13a et sur un axe perpendiculaire du plateau 13.

Ledit pénétromètre 26 se compose d'un poinçon 27 dont l'extrémité 27a (figure 2a) forme une concavité délimitée par une arête circulaire coupante $27a_1$, dont la fonction est de couper la peau du fruit avant de pénétrer dans la chair. Ledit poinçon 27 est situé au-dessus du plateau 13 et est fixé à l'extrémité d'un capteur de force 28, lui-même fixé à la tige mobile 29a d'un vérin à double effet 29, lequel poinçon est monté coulissant dans un trou 30a d'une coupelle 30 de centrage du fruit 25, solidaire du bâti 1 de l'unité robotisée.

Ledit poinçon 27 est coaxial à une coupelle mobile 31, d'un diamètre inférieur à celui des trous 13a du plateau 13. La coupelle mobile 31 est située au-dessous dudit plateau 13 et est montée sur la tige mobile 32a d'un vérin à double effet 32, lui-même actionné par un second vérin 33 à course plus réduite.

Le vérin 32 est fixé à l'extrémité de la tige mobile 33a dudit vérin 33.

Les deux faces concaves en vis à vis des coupelles 30/31, permettent de centrer le fruit sur l'axe du poinçon 27 du pénétromètre 26.

Le processus de mesure de la dureté des fruits est le suivant :

Après avoir été pesé, le fruit 25 calé dans le trou 13a du plateau 13, se présente en synchronisme avec les phases précédentes à la verticale du pénétromètre 26. Le verrou de blocage 19 stoppe le plateau 13. Le vérin 32 est actionné, le fruit 25 est soulevé par la coupelle inférieure 31, jusqu'à ce qu'il soit mis au contact de la coupelle supérieure fixe 30. Il reste inséré entre les deux coupelles 30/31 tout le temps de la mesure de dureté.

Le vérin supérieur 29 est actionné pour descendre le poinçon 27 et le faire pénétrer dans le fruit 25 sous une poussée et sur une profondeur constantes.

Pendant la pénétration du poinçon 27 dans le fruit, le capteur de force 28 mesure la force avec laquelle la chair du fruit s'oppose à la pénétration et cette force est mémorisée dans l'ordinateur 2. On en déduit ensuite la dureté du fruit par comparaison de la force mémorisée et de paramètres stockés dans la mémoire de l'ordinateur 2. Leur moyenne est calculée en fin de lot d'échantillonnage.

Après mémorisation de la mesure de dureté, les vérins supérieur 29 et inférieur 32 sont remis à leur position initiale de repos.

L'unité comporte au droit de l'axe sur lequel se déplace le poinçon 27 du pénétromètre, un éjecteur 34 du fruit 25 venant d'être testé.

Ledit éjecteur 34 se compose d'un vérin à double effet 34a disposé horizontalement, parallèlement au plateau 13, dont la tige mobile porte une palette-poussoir 34b.

Après mémorisation de la mesure de dureté et après que les vérins 32/29 soient remis dans leur position initiale, le vérin 33 est actionné pour maintenir le fruit au-dessus du plateau 13.

Le vérin éjecteur 34 est actionné et le fruit est éjecté à l'intérieur du plateau annulaire vers des moyens pour extraire le jus des fruits. Lesdits moyens sont constitués par un pressoir 35 disposé horizontalement au-dessous du plateau 13 et comprenant un cylindre 36 ouvert à ses deux extrémités et comportant une ouverture latérale 36a réservée dans sa paroi. Cette ouverture 36a est située à la partie du cylindre la plus rapprochée du plateau 13. Le diamètre interne du cylindre 36 et le diamètre de ladite ouverture 36a sont supérieurs à celui des plus gros fruits à analyser.

Les fruits 25 issus du plateau 13 et éjectés par le dispositif 34 tombent par gravité dans ledit cylindre, en passant par l'ouverture latérale 36a.

Dans ledit cylindre 36 se déplace un piston 37, fixé à la tige mobile 38a d'un vérin à double effet 38. Ledit vérin 38 est coaxial au cylindre 36 et monté solidaire du bâti par tous moyens connus. Son extrémité ouverte 36b située à l'opposé dudit vérin 38 est en regard d'une enclume 39, par exemple cylindrique, laquelle est coaxiale audit cylindre 36, et montée au bâti de l'unité robotisée.

Ledit pressoir 35 comporte des moyens pour rapprocher ou éloigner le cylindre 36 de l'enclume 39. Ces moyens consistent en un vérin à double effet 40 fixé audit bâti et disposé parallèlement et au-dessous du cylindre 36. Sa tige mobile est reliée à une patte 36c fixée à proximité de l'extrémité ouverte 36b et à l'opposé de l'ouverture 36a.

L'enclume 39 forme une cuvette sur sa face située en regard du cylindre 36 et comporte à son point le plus bas un orifice auquel est relié un tube collecteur du jus 41.

En position initiale du pressoir, la tige du vérin du cylindre 40 est sortie et celle du vérin 38 est rentrée. Dans ces conditions, ledit cylindre 36 est obturé en étant en appui sur l'enclume 39. L'ouverture 36a est libre de recevoir un fruit.

Après avoir subi la mesure de dureté, le fruit 25 est éjecté du plateau 13 et tombe par gravité dans le cylindre 36 par ladite ouverture 36a. Le vérin de piston 38 est actionné, le fruit 25 est déplacé par le piston 37, jusqu'à l'enclume 39 et est ensuite écrasé entre ledit piston et l'enclume.

Le jus résultant de cette pression s'écoule par le tube collecteur 41. Après le temps nécessaire à l'extraction totale du jus, le vérin de cylindre 40 est actionné simultanément au vérin de piston 38. Les deux tiges des vérins 38/40 sont rentrées, le cylindre 36 s'écarte de l'enclume 39 et la matière sèche du fruit écrasé 25a tombe par gravité dans la poubelle 12.

Le vérin 40 est ensuite actionné et remet le cylindre 36 en appui sur l'enclume 39 en attente du fruit suivant.

Le taux de sucre de chaque fruit est mesuré pendant l'écoulement de son jus extrait au moyen du pressoir 35. La tubulure 41 est inclinée et le jus issu de l'enclume 39 s'écoule par gravité à travers un réfractomètre 42, jusqu'aux moyens de mesure d'acidité qui seront décrits plus loin.

Les mesures du taux de sucre de chaque fruit sont mémorisées, puis leur moyenne est calculée à la fin du lot d'échantillonnage.

La définition du rapport $\frac{\text{poids total des fruits}}{\text{poids total du jus}}$ nécessite la collecte totale du jus extrait. La mesure d'acidité nécessite une collecte partielle du jus de chaque fruit pour limiter la consommation de soude et le temps d'analyse.

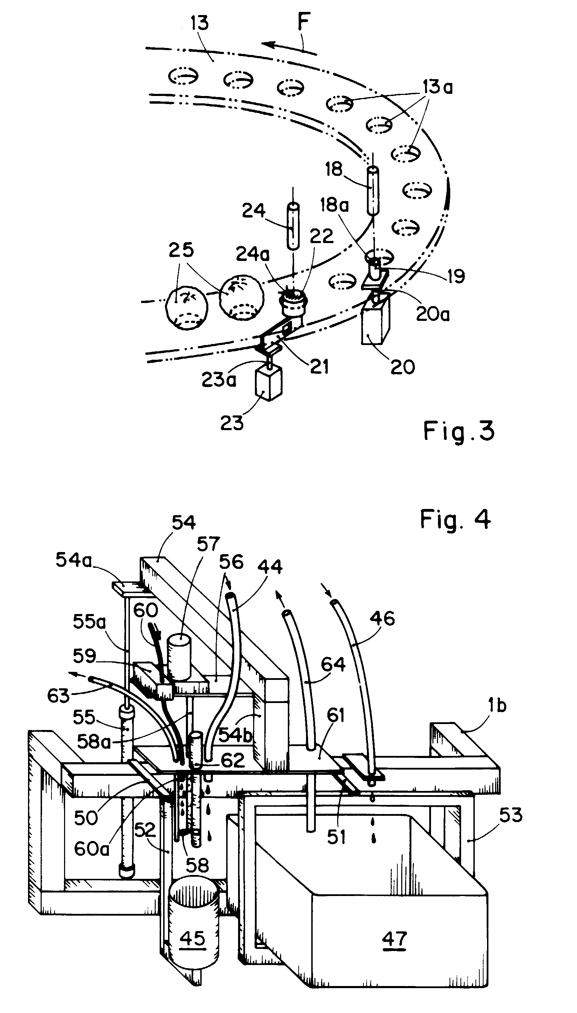

Pour réaliser ces mesures, l'unité comporte un té 43 incliné, par exemple à 45°, dont le tube principal 43a est relié par ses extrémités à deux tronçons de ladite tubulure 41 et dont le tube de dérivation 43b qui est situé au-dessous dudit tube principal 43a, est raccordé à une seconde tubulure souple 44, aboutissant au-dessus d'un bac de récolte partielle 45 (fig.4).

Le jus issu du réfractomètre 42 s'écoule par une tubulure 46 et est recueilli dans un autre bac de réception 47 (figure 4), dit bac de collecte totale.

En aval du té 43 est montée une électrovanne 48 du type à pincement de tube. En aval de ladite électrovanne 48, la tubulure 44 est enroulée en spirale 44a en formant par exemple trois spires.

En aval de ladite spirale, la tubulure 44 comporte une seconde électrovanne 49 identique à celle 48 susmentionnée. La spirale 44a constitue une réserve de jus, lorsque l'électrovanne 49 est fermée.

Initialement, les électrovannes 48/49 sont fermées. Le jus du fruit extrait par le pressoir 35 s'écoule par gravité par le tube principal 43a du té et remplit le tube de dérivation 43b et le tronçon de la tubulure 44 fermée par l'électrovanne 48, puis le trop-plein s'écoule à travers le réfractomètre 42 jusqu'audit bac 47.

A la fin de l'extraction du jus, l'électrovanne 48 est ouverte et la quantité de jus contenue dans le tube de dérivation 43b et le tronçon de tube 44 s'écoule dans la réserve de jus que constitue la spirale 44a.

L'électrovanne 48 est fermée jusqu'au fruit suivant. A la fin du lot d'échantillonnage, le bac de collecte totale 47 contient la quasi-totalité du jus. L'électrovanne 49 est alors ouverte pour déverser dans le bac de récolte partielle 45 la totalité du jus contenue dans la spirale 44a et représentant la somme des échantillons calibrés de chaque fruit dudit lot.

Les différents postes fonctionnant de façon simultanée, le dispositif de réserve de jus permet d'effectuer la mesure d'acidité du lot en cours, pendant qu'un nouveau lot est introduit dans l'unité, en vue de la mesure du poids de dureté de taux de sucre...

Le poids total de jus est égal à la somme des poids de jus dans les deux bacs de réception de récolte partielle 45 et de récolte totale 47.

Les mesures de poids sont réalisées au moyen des capteurs de force 50/51.

Le bac 45 est monté sur un support 52 fixé à l'extrémité libre du capteur de force 50, lequel est fixé par son autre extrémité au bâti 1b. Le bac 47 est monté sur un cadre-support 53 fixé à l'extrémité libre du capteur de force 51, dont l'autre extrémité est fixée audit bâti 1b.

Pour la mesure de l'acidité, le principe retenu est le titrage par réaction acido-basique. La base utilisée est de la soude diluée.

Le dispositif de titrage automatique est porté par un bras 54 déplaçable verticalement au moyen d'un vérin à double effet 55 fixé au bâti 1b et dont la tige mobile 55a est fixée à une patte 54a solidaire dudit bras. Celui-ci est bien entendu guidé en translation verticale par des moyens de glissière (non représentés). Il comporte une première platine-support 56 s'étendant horizontalement et sur laquelle est monté un moto-réducteur 57 sur l'arbre de sortie duquel est calé l'axe 58a d'un agitateur 58 disposé verticalement.

La platine 56 porte également une électrovanne à pincement de tube 59, dont le tube 60 est relié d'une part; à une réserve de soude (non représentée) et d'autre part, a son extrémité basse 60a située au-dessous de la platine 56 et en regard du bac de réception partielle 45.

Le bras 54 comporte en outre un prolongement vertical 54b s'étendant vers le bas, à l'extrémité duquel est fixée une seconde platine 61 parallèle à ladite platine 56. Cette seconde platine 61 porte une sonde de pH 62, s'étendant verticalement et parallèlement à l'axe de l'agitateur 58.

Le tube 60 d'arrivée de la soude et le tube 44 de collecte partielle aboutissent à cette platine et le dépassent légèrement par le dessous.

A cette platine, aboutit également un tube d'aspiration 63, pour vider le bac 45 à la fin de l'analyse d'un lot et un autre tube d'aspiration 64, pour vider le bac de récolte totale 47.

L'axe de l'agitateur 58 traverse la platine 61. L'agitateur 58, la sonde de pH 62 et les tubes 44/60/63 sont regroupés pour se présenter au-dessus du bac de réception 45. En position rentrée de la tige 55a du vérin 55, le bras 54 est descendu et lesdits tubes 44/60/63, la sonde 62 et l'agitateur 58 sont introduits dans le récipient 45. Parallèlement, le tube d'aspiration 64 est introduit dans le bac de récolte totale 47.

En position sortie de la tige 55a dudit vérin, le bras s'immobilise en position haute, ce qui a pour effet de permettre de dégager manuellement les deux bacs 45/47, pour en assurer le nettoyage en fin de journée.

Pendant les mesures, le bras 54 est en position basse, la tige de vérin 55 est rentrée.

Pendant toute la durée d'extraction du jus des fruits, l'électrovanne 59 est fermée, et l'agitateur 58 est à l'arrêt.

Lorsque l'échantillonnage du jus est terminé, le contenu de la spirale 44a ayant été vidé dans le bac de réception partielle 45, le poids de jus est mesuré par le capteur de force 50, puis mémorisé. L'agitateur est mis en marche et le pH est mesuré au moyen de la sonde 62 puis mémorisé après stabilisation de la mesure. L'électrovanne de soude 59 est ensuite ouverte jusqu'à ce que la valeur de pH atteigne un seuil prédéterminé et mise en mémoire dans l'ordinateur 2, par exemple pH = 6.

A pH=6, l'ouverture de l'électrovanne 59 est modulée à une cadence de plus en plus faible, au fur et à mesure que le pH augmente.

A pH=8,2 par exemple, l'électrovanne 59 est fermée. Le poids de la solution est alors de nouveau mesuré au moyen du capteur de force 50, puis mémorisé. L'agitateur 58 est stoppé. L'acidité est calculée en fonction du poids initial du jus et de la quantité de soude ajoutée pour obtenir le pH 8,2 (valeur correspondant à l'équivalence de la réaction acido-basique).

En fin de mesure, les deux bacs 45/47 sont vidés par aspiration au moyen des tubes 63/64, lesquels sont reliés à un convergent/divergent de venturi (non représenté).

En fin de lot d'échantillonnage, l'ordinateur analyse les différents paramètres, tels que l'indice de réfraction, la dureté, l'acidité, le rapport poids/jus, afin d'apprécier :

- la qualité gustative,
- les possibilités de conservation,
- l'aptitude à la transformation industrielle (jus de fruits, fruits au sirop, confitures, etc...).

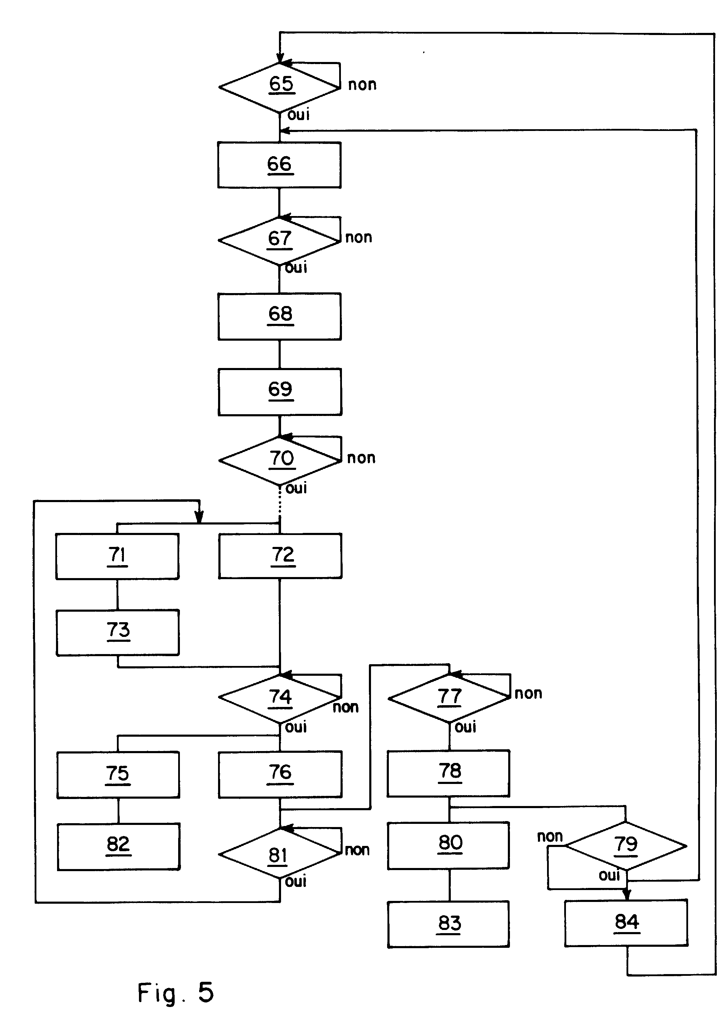

Le diagramme de fonctionnement de l'unité est donné à la figure 5 sous la forme d'un schéma-bloc.

Les différents postes de l'unité robotisée fonctionnent par phases successives simultanées. Ainsi, après le pesage du premier fruit, le pesage du fruit suivant s'effectue pendant la mesure de dureté du fruit précédent. Le pesage du troisième fruit s'effectue pendant la mesure de dureté du deuxième fruit, simultanément à la pression du premier fruit, en vue d'en extraire le jus, etc...

La mesure de l'acidité s'effectue pendant les phases précédentes.

Le diagramme de la figure 5 donne le fonctionnement de l'unité robotisée selon l'invention.

Les fruits d'un lot d'échantillonnage sont sélectionnés et placés sur le plateau annulaire 13 en 65.

En 66, rotation du plateau afin d'amener le premier fruit au poste de pesage.

En 67, le plateau étant toujours en rotation, attente que la condition : "le premier fruit est au pesage" soit réalisée.

En 68, pesage premier fruit.

En 69, rotation du plateau.

En 70, le plateau étant en rotation, attente que la condition : "premier fruit sous le pénétromètre 26" soit réalisée.

En 71-72 pesage du deuxième fruit simultané à la mesure de la dureté du premier fruit ou pesage du N+1 fruit et mesure de la dureté du Nième fruit..

En 73, éjection du 1er ou du Nième fruit

En 74, attente que la condition : "Nième fruit pressé + N + 1 fruit pesé" soit réalisée.

En 75, le Nième fruit est pressé. Prélèvement d'une dose de jus et stockage temporaire dans la réserve en spirale 44a.

Parallèlement à l'étape 75, en 76 rotation du plateau annulaire 13.

En 81-77, attente que l'une des deux conditions : "N + 1 fruit sous le pénétromètre" (81) et "Fin de lot" (77) soit réalisée.

- Si (81) est réalisée, le cycle reboucle aux étapes 71 et 72 pour traiter les fruits suivants.
- Si (77) est réalisée, c'est-à-dire que le N + 1 fruit était le dernier du lot et que l'opérateur a envoyé l'ordre "Fin de lot" par l'intermédiaire de l'ordinateur, le cycle se déroute vers l'étape 78.

En 82, mesure du taux de sucre du jus du Nième fruit.

En 83, rinçage du dispositif acidité au terme de l'étape 80.

Bien entendu, sans sortir du cadre de l'invention, les parties qui viennent d'être décrites à titre d'exemple, pourront être remplacées par l'homme du métier par des parties équivalentes remplissant la même fonction.

**Revendications**

1. Procédé pour analyser automatiquement les paramètres physico-chimiques sur un échantillonnage de fruits (25), au moyen d'une unité robotisée (1) comprenant un automate programmable (6) que l'on relie à un ordinateur (2), caractérisé par les étapes suivantes :

   a) on réceptionne sur des moyens récepteurs à défilement pas à pas (13) les fruits d'un lot d'échantillonnage ;

b) on pèse (21) chaque fruit dudit échantillonnage et on entre les mesures pondérales dans la mémoire dudit ordinateur (2) ;

c) on mesure (26) la dureté desdits fruits et on entre lesdites mesures dans la mémoire dudit ordinateur (2) ;

d) on extrait (35) le jus desdits fruits de l'échantillonnage ;

e) on mesure le taux de sucre contenu dans lesdits fruits au moyen d'un réfractomètre électronique (42) et cn entre lesdites mesures dans la mémoire dudit ordinateur (2) ;

f) on collecte la totalité du jus (47) extrait précédemment ;

g) on pèse la totalité du jus extrait et cn entre cette mesure dans la mémoire dudit ordinateur (2) ;

h) on collecte parallèlement à l'étape f) une partie dudit jus extrait (44a) ;

i) on mesure l'acidité de ladite partie du jus extrait du lot d'échantillonnage par réaction acido-basique et on entre cette mesure dans la mémoire dudit ordinateur (2) ;

lequel ordinateur (2) donne le résultat des analyses et les qualités organoleptiques des fruits déterminées par :

- le rapport $\dfrac{\text{poids total des fruits}}{\text{poids total du jus extrait}}$,
- la dureté moyenne des fruits,
- le taux moyen de sucre,
- l'acidité moyenne des fruits.

2. Unité robotisée (1) pour l'analyse des paramètres physico-chimiques d'un échantillonnage de fruits (25), comprenant un automate programmable (6) relié à un ordinateur (2) et des moyens de commande des énergies nécessaires (7) au fonctionnement de ladite unité, caractérisée en ce qu'elle comporte en combinaison :

a) des moyens récepteurs (13) de défilement pas à pas des fruits (25) d'un lot d'échantillonnage ;

b) des moyens (21/22) pour peser chaque fruit dudit échantillonnage ;

c) des moyens (26) pour mesurer la dureté desdits fruits ;

d) des moyens (35) pour extraire le jus desdits fruits ;

e) des moyens (42) pour mesurer le taux de sucre contenu dans le jus extrait ;

f) des moyens (46/47) pour collecter la totalité du jus extrait ;

g) des moyens (51) pour peser la totalité du jus extrait ;

h) des moyens (43/44a) pour collecter une partie dudit jus extrait ;

i) des moyens (58/59/62) pour mesurer l'acidité de ladite partie du jus extrait du lot d'échantillonnage et en ce que les mesures effectuées en b), c), e), g) et i) sont entrées dans la mémoire dudit ordinateur (2), lequel compare lesdites mesures et les paramètres entrés dans sa mémoire et donne les résultats de l'analyse et les qualités organoleptiques des fruits :

- le rapport $\dfrac{\text{poids total des fruits}}{\text{poids total du jus extrait}}$,
- la dureté moyenne des fruits,
- le taux moyen de sucre,
- l'acidité moyenne des fruits.

3. Unité robotisée selon la revendication 2 comportant un bâti (1bj et des moyens de commande des énergies nécessaires (6/7) au fonctionnement de ladite unité, caractérisée en ce qu'elle comporte un plateau (13) sensiblement horizontal, monté à rotation sur ledit bâti (1b) et comportant des orifices circulaires (13a) régulièrement répartis sur le périmètre du plateau, lequel est entraîné séquentiellement par un moto-réducteur (16) par fraction de tour correspondant à l'arc qui sépare deux orifices (13a), lesquels sont d'un diamètre inférieur à celui des fruits (25), de telle sorte que les fruits placés sur ledit plateau, demeurent partiellement engagés dans lesdits orifices en attente de leur analyse.

4. Unité selon l'une quelconque des revendications 2 et 3, caractérisée en ce que ledit plateau (13) adopte une forme annulaire et est monté tournant sur des galets (14) montés à rotation libre, sur ledit bâti, lequel plateau est soumis à l'action de galets de centrage (15) en appui sur son bord périphérique et que son entraînement en rotation est réalisé par un galet de friction (15a) soumis à l'action de moyens élastiques (17) et calé sur l'arbre de sortie dudit moto-réducteur séquentiel (16).

5. Unité selon l'une quelconque des revendications 3 et 4, caractérisée en ce que ledit plateau (13) comporte un détecteur (18) de présence des orifices (13a), associé à un doigt d'arrêt escamotable (19) situé sur le trajet desdits orifices, lequel doigt est d'une section inférieure à celle desdits trous (13a), pour y pénétrer à l'intérieur et stopper séquentiellement le plateau (13) dans une position précise.

6. Unité selon l'une quelconque des revendications 2 à 5, caractérisée en ce que les moyens pour peser chaque fruit se composent d'un détecteur (24) de présence des fruits, associé à une coupelle escamotable (22) d'un diamètre inférieur à celui des orifices (13a) du plateau, et situé sur le trajet desdits orifices, laquelle coupelle (22) est portée par un capteur de force (21) relié audit ordinateur (2) et est soumise à l'action de moyens (23) pour la faire pénétrer dans lesdits orifices, afin de soulever séquentiellement et en synchronisme avec le fonctionnement du plateau (13) un fruit de l'échantillonnage en vue de sa pesée.

7. Unité selon l'une quelconque des revendications 2 à 6, caractérisée en ce que les moyens pour mesurer la dureté des fruits, se composent de moyens (30/31/32) pour immobiliser chaque fruit séquentiellement en synchronisme avec les autres organes de l'unité, et d'un pénétromètre (26) accouplé à un capteur de force (28), relié audit ordinateur (2), dont le poinçon (27) est actionné en synchronisme pour pénétrer dans le fruit.

8. Unité selon la revendication 7, caractérisée en ce que les moyens pour mesurer la dureté des fruits, se composent d'une première coupelle fixe (30) solidaire du bâti (1b), située au-dessus du plateau et sur le trajet des orifices (13a), au droit de laquelle s'immobilisent successivement les orifices du plateau (13) et une seconde coupelle (31) escamotable, coaxiale à ladite première coupelle (30), dont les concavités desdites coupelles (30/31) sont en vis à vis, en ce que ladite seconde coupelle (31) est d'un diamètre inférieur à celui desdits orifices (13a) et est actionnée par des moyens télescopiques (32/33) situés au-dessous dudit plateau (13), pour lui faire traverser tour à tour lesdits orifices (13a) et amener le fruit (25), quelle que soit sa grosseur, en appui sur ladite première coupelle (30), laquelle comporte un trou central (30a) à travers lequel passe le poinçon (27) du pénétromètre (26).

9. Unité selon l'une quelconque des revendications 2 à 8, caractérisée en ce qu'elle comporte au droit de l'axe du pénétromètre (26) un éjecteur (34) des fruits ayant passé le poste de mesure de dureté (26), lequel éjecteur (34) est parallèle audit plateau (13) et est situé entre ledit plateau et ladite première coupelle fixe (30) du pénétromètre.

10. Unité selon l'une quelconque des revendications 8 et 9, caractérisée en ce que lesdits moyens télescopiques de la seconde coupelle (31) se composent de deux vérins (32/33) superposés, dont l'un (32) a pour fonction de déplacer ladite coupelle (31) pour mettre le fruit en appui sur ladite première coupelle fixe (30), et dont l'autre vérin (33) a pour fonction d'immobiliser momentanément la coupelle (31) au-dessus dudit plateau (13), pour mettre le fruit sensiblement dans le champ d'action dudit éjecteur (34) et transférer le fruit : dudit plateau (13) jusqu'audit moyen d'extraction du jus (35).

11. Unité selon l'une quelconque des revendications 7 à 10, caractérisée en ce que l'extrémité (27a) du poinçon (27) du pénétromètre (26) comporte une concavité (27a₁) délimitée par un bord périphérique affûté, pour trancher la peau du fruit préalablement à la pénétration du poinçon dans la chair du fruit.

12. Unité selon l'une quelconque des revendications 2 à 11, caractérisée en ce que lesdits moyens pour extraire le jus des fruits, sont constitués par un pressoir (35), disposé sensiblement horizontalement et au-dessous dudit plateau (13), lequel pressoir comporte un orifice latéral (36a) pour recevoir les fruits (25) éjectés du plateau (13).

13. Unité selon la revendication 12, caractérisée en ce que ledit pressoir (35) se compose d'un cylindre (36) ouvert à ses extrémités, dont la paroi comporte une ouverture (36a) pour l'introduction des fruits (25) par gravité, dans lequel cylindre est monté un piston (37) fixé à la tige mobile (38a) d'un vérin à double effet (38) pour déplacer le piston (37) sur la longueur dudit cylindre, lequel cylindre (36) est attelé à la tige mobile d'un vérin à double effet (40), dont le corps est fixé au bâti (1b) et dont la tige a pour fonction d'appuyer le cylindre (36) contre une enclume (39) fixée audit bâti, et pour l'éloigner de celle-ci, afin de laisser tomber par gravité la matière sèche du fruit après extraction du jus, sous la pression exercée par ledit piston (37) sur le fruit en appui sur l'enclume (39), à laquelle enclume est fixée une tubulure d'écoulement du jus (41).

14. Unité selon l'une quelconque des revendications 2 à 13, dont le jus issu de ladite enclume (39) est canalisé par une première tubulure (41) pour passer à travers un réfractomètre (42) en vue de mesurer le taux de sucre, et ensuite amener le jus dans un bac de réception dit de collecte totale (47), caractérisée en ce qu'elle comporte en amont dudit réfractomètre (42) un té (43), dont le tube principal (43a) raccordé à ladite première tubulure (41) est incliné en aval vers le réfractomètre (42) et dont le tube de dérivation (43b) est situé au-dessous dudit tube principal et raccordé à une seconde tubulure (44) aboutissant à un second bac de réception dit de collecte partielle, laquelle seconde tubulure (44) comporte une première vanne (48) située du côté dudit té (43) pour recueillir, en position fermée de la vanne, un échantillon du jus de chaque fruit du lot et une seconde vanne (49) située en aval de ladite première vanne pour recueillir en position fermée de ladite seconde vanne la totalité des échantillons du jus des fruits d'un lot d'échantillonnage.

15. Unité selon la revendication 14, caractérisée en ce que lesdits bacs de réception de récolte totale (47) et de récolte partielle (45) sont chacun portés par un capteur de force (50/51) fixé audit bâti et relié audit ordinateur (2).

**16.** Unité selon l'une quelconque des revendications 14 et 15, caractérisée en ce qu'elle comporte une tubulure (60) issue de la réserve d'une base et aboutissant dans ledit second bac de réception de collecte partielle (45), sur laquelle tubulure est insérée une vanne (59) reliée à des moyens de commande pour délivrer dans ledit bac (45) des doses de ladite base ; un agitateur (58) et une sonde de Ph (62) reliée audit ordinateur (2).

**17.** Unité selon l'une quelconque des revendications 14 à 16, caractérisée en ce que ladite vanne de dosage (59) de la base, ledit agitateur (58) et ladite sonde (62) sont montés sur un bras (54) déplaçable verticalement et actionné par un vérin à double effet (55), pour plonger la sonde (62) et l'agitateur (58) dans ledit bac de récolte partielle (45) ou pour les extraire dudit bac, afin de permettre le retrait des bacs de récolte totale (47) et de récolte partielle (45) en vue de leur nettoyage périodique.

**Claims**

**1.** A method of automatically analyzing the physico-chemical parameters of a sample batch of fruit (25) by means of a robotic unit (1) comprising a programmable controller (6) connected to a computer (2), the method being characterized by the following steps:

a) the fruit of a sample batch are received on receiver means (13) that travel stepwise;
b) each fruit of said batch is weighed (21) and the measured weights are entered into the memory of said computer (2);
c) the hardness of said fruit is measured (26) and said measurements are entered into the memory of said computer (2);
d) juice is extracted (35) from the fruit of the batch;
e) the sugar content of said fruit is measured by means of an electronic refractometer (42) and said measurements are entered into the memory of said computer (2);
f) all of the previously extracted juice (47) is collected;
g) all of the extracted juice is weighed and said measurement is entered into the memory of said computer (2);
h) in parallel with step f), a portion of said extracted juice (44a) is collected;
i) the acidity of said portion of the juice extracted from the sampling batch is measured by tne acid-base reaction and said measurement is entered into the memory of said computer (2);

which computer (2) gives the result of the analyses and the organoleptic qualities of the fruit, determined as follows:

- the ratio $\dfrac{\text{total weight of fruit}}{\text{total weight of extracted juice}}$;
- the mean hardness of the fruit;
- the mean sugar content; and
- the mean acidity of the fruit.

**2.** A robotic unit (1) for analyzing the physico-chemical parameters of a sample batch of fruit (25), comprising a programmable controller (6) connected to a computer (2) and means for controlling the energy (7) required for operation of said unit, characterized in that it comprises in combination:

a) receiver means (13) that travel stepwise for receiving the fruit (25) of a sample batch;
b) means (21/22) for weighing each fruit of said batch;
c) means (26) for measuring the hardness of said fruit;
d) means (35) for extracting the juice of said fruits;
e) means (42) for measuring the sugar content of the extracted juice;
f) means (46/47) for collecting all of the extracted juice;
g) means (51) for weighing all of the extracted juice;
h) means (43/44a) for collecting a portion of said extracted juice; and
i) means (58/59/62) for measuring the acidity of said portion of the juice extracted from the sampling batch, and in that the measurements performed at b), c), e), g), and i) are entered into the memory of said computer (2), which compares said measurements with the parameters entered in its memory, and gives the results of the analysis and the organoleptic qualities of the fruit, as follows:

- the ratio $\dfrac{\text{total weight of fruit}}{\text{total weight of extracted juice}}$;
- the mean hardness of the fruit;
- the mean sugar content; and
- the mean acidity of the fruit.

**3.** A robotic unit according to claim 2, comprising a support structure (1b) and means (6/7) for controlling the energy required for operation of said unit, characterized in that it includes a substantially horizontal turntable (13) mounted to rotate on said support structure (1b) and including circular orifices (13a) regularly distributed around the perimeter of the turntable, which turntable is driven sequentially by a motor and gearbox unit (16) through a fraction of a revolution corresponding to the arc between two orifices (13a), which orifices are of a diameter smaller than the diameter of the fruit (25) such that the fruit placed on said turntable remain partially engaged in said orifices while waiting to be analyzed.

4. A unit according to claim 2 or 3, characterized in that said turntable (13) is annular in shape and is mounted to rotate on said support structure via freely rotatable wheels (14), which turntable is subjected to the action of centering wheels (15) bearing against its periphery, and is driven in rotation by a friction wheel (15a) subjected to the action of resilient means (17) and constrained to rotate with the outlet shaft of said sequential motor and gearbox unit (16).

5. A unit according to claim 3 or 4, characterized in that said turntable (13) includes a detector (18) for detecting the presence of the orifices (13a), associated with a retractable stop finger (19) situated on the path of said orifices, which finger is of section smaller than the section of said holes (13a) to penetrate therein and stop the turntable (13) sequentially in an accurate position.

6. A unit according to any one of claims 2 to 5, characterized in that the means for weighing each fruit comprise a detector (24) for detecting the presence of a fruit and an associated retractable cup (22) of diameter smaller than that of the orifices (13a) of the tray and situated on the path of said orifices, which cup (22) is carried by a force sensor (21) connected to said computer (2) and is subject to the action of means (23) for causing it to penetrate into said orifices to raise each fruit of the batch sequentially and synchronously with the operation of the turntable (13) in order to weigh the fruit.

7. A unit according to any one of claims 2 to 6, characterized in that the means for measuring the hardness of the fruit comprise means (30/31/32) for immobilizing each fruit sequentially and synchronously with the other members of the unit, and a penetration meter (26) coupled to a force sensor (28) connected to said computer (2), the punch (27) thereof being actuated synchronously to penetrate into the fruit.

8. A unit according to claim 7, characterized in that the means for measuring the hardness of the fruit is made up of a fixed first cup (30) secured to the support structure (1b) situated above the turntable and on the path of the orifices (13a) with the orifices of the turntable (13) coming to rest in succession in register therewith, and a retractable second cup (31) on the same axis as said first cup (30), the concave sides of said cups (30/31) facing each other, and in that said second cup (31) is of a diameter smaller than the diameter of said orifices (13a) and is actuated by telescopic means (32/33) situated beneath said turntable (13) to cause it to pass through said orifices (13a) in turn and to bring the fruit (25) whatever its size to bear against said first cup (30), which includes a central hole (30a) through which the punch (27) of the penetration meter (26) passes.

9. A unit according to any one of claims 2 to 8, characterized in that it includes an ejector (34) at right angles to the axis of the penetration meter (26) to eject fruit that has passed the station for measuring hardness (26), said ejector (34) being parallel to said turntable (13) and being situated between said turntable and said fixed first cup (30) of the penetration meter (26).

10. A unit according to claim 8 or 9, characterized in that said telescopic means of the second cup (31) are composed by two superposed actuators (32/33), one of the actuators (32) having the function of moving said cup (31) to bring the fruit to bear against said fixed first cup (30), and the other actuator (33) having the function of momentarily holding the second cup (31) stationary above said turntable (13) to put the fruit substantially into the field of action of said ejector (34) to transfer the fruit from said turntable (13) to said juice extraction means (35).

11. A unit according to any one of claims 7 to 10, characterized in that the end (27a) of the punch (27) of the penetration meter (26) includes a concave portion ($27a_1$) defined by a sharp peripheral edge, to cut through the skin of the fruit prior to the punch penetrating into the flesh of the fruit.

12. A unit according to any one of claims 2 to 11, characterized in that said means for extracting the juice of the fruit are constituted by a press (35) disposed substantially horizontally and beneath said turntable (13), which press has a lateral orifice (36a) for receiving the fruit (25) ejected from the turntable (13).

13. A unit according to claim 12, characterized in that said press (35) comprises a cylinder (36) that is open at its ends, the wall of the cylinder having an opening (36a) through which the fruit (25) are inserted under gravity, said cylinder having a piston (37) mounted therein, the piston being fixed to the moving rod (38a) of a double-acting actuator (38) to move the piston (37) along said cylinder, which cylinder (36) is coupled to the moving rod of a double-acting actuator (40) whose cylinder is fixed to the support frame (1b) and whose rod has the function of pressing the cylinder (36) against an anvil (39) fixed to said support structure, and for moving it away therefrom so as to allow the dry matter of the fruit to fall away under gravity after the juice has been extracted under the pressure exerted by said piston (37) on the fruit pressed against the anvil (39), which anvil has a juice flow hose (41) fixed thereto.

14. A unit according to any one of claims 2 to 13, in which the juice from said anvil (39) is channeled by a first hose (41) to pass through a refractometer (42) in order to measure its sugar content, and thereafter to bring the juice into a "total collection" receiver tank (47), the unit being characterized in that upstream from said refractometer (42) it includes a tee (43) having its main tube (43a) connected to said first hose (41) and sloping downstream towards the refractometer (42), and whose branch tube (43b) is situated beneath said main tube and is connected to a second hose (44) terminating at a "partial collection" second receiver tank, which second hose (44) includes a first valve (48) situated beside said tee (43) and organized, when the valve is in the closed position, to collect a sample of the juice from each fruit of the batch, and a second valve (49) situated downstream from the first valve and organized, when the second valve is in the closed position, to collect all of the samples of juice from the fruit of a sample batch.

15. A unit according to claim 14, characterized in that each of said total collection receiver tank (47) and said partial collection tank (45), is carried by a respective force sensor (50/51) fixed to said support structure and connected to said computer (2).

16. A unit according to claim 14 or 15, characterized in that it includes a hose (60) coming from a supply of a base and terminating in said partial collection second receiver tank (45), said hose having a valve (59) inserted therein and connected to control means for delivering measured quantities of said base into said tank (45); a stirrer (58), and a pH probe (62) connected to said computer (2).

17. A unit according to any one of claims 14 to 16, characterized in that said valve (59) for measuring out the base, said stirrer (58), and said probe (62) are mounted on an arm (54) that is vertically movable and actuated by a double-acting actuator (55), in order to dip the probe (62) and the stirrer (58) into said partial collection tank (45) or to extract them from said tank, thereby enabling the total and partial collection tanks (47, 45) to be removed for periodic cleaning.

**Patentansprüche**

1. Verfahren zur automatischen Analyse der physikalisch-chemischen Parameter einer Stichprobe von Früchten (25) mittels einer automatisierten Einheit (1), die einen programmierbaren Automaten (6) aufweist, den man mit einem Computer (2) verbindet, gekennzeichnet durch die folgenden Schritte:

a) die Früchte eines Stichprobenloses werden auf schrittweise vorbeilaufende Aufnahmemitteln (13) aufgebracht;
b) jede Frucht dieser Stichprobe wird gewogen (21), und die Meßwerte werden in den Speicher des Computers (2) eingegeben;
c) die Härte der Früchte wird gemessen (26), und die Meßwerte werden in den Speicher des Computers (2) eingegeben;
d) der Saft wird aus den Früchten der Stichprobe entzogen (35);
e) der Gehalt des in den Früchten enthaltenen Zuckers wird mittels eines elektronischen Refraktometers (42) gemessen, und diese Meßwerte werden in den Speicher des Computers (2) eingegeben;
f) die Gesamtheit des vorher entzogenen Safts wird gesammelt (47);
g) die Gesamtheit des entzogenen Safts wird gewogen, und dieser Meßwert wird in den Speicher des Computers (2) eingegeben;
h) parallel zum Schritt f) wird eine Teilmenge des entzogenen Safts gesammelt (44a);
i) der Säuregehalt dieser Teilmenge des aus dem Stichprobenlos entzogenen Safts wird durch saure und basische Reaktion gemessen, und dieser Meßwert wird in den Speicher des Computers (2) eingegeben;

der Computer (2) gibt die Ergebnisse der Analyse und die organoleptischen Eigenschaften der Früchte an, die bestimmt werden durch:

- das Verhältnis Gesamtgewicht der Früchte/Gesamtgewicht des entzogenen Safts,
- die mittlere Härte der Früchte,
- den mittleren Zuckergehalt,
- den mittleren Säuregehalt der Früchte.

2. Automatisierte Einheit (1) zur Analyse der physikalischchemischen Parameter einer Stichprobe von Früchten (25), die einen mit einem Computer (2) verbundenen, programmierbaren Automaten (6) und Mittel (7) zur Steuerung der für den Betrieb dieser Einheit notwendigen Energien aufweist, dadurch gekennzeichnet, daß sie in Kombination enthält:

a) schrittweise vorbeilaufende Mittel (13) zur Aufnahme der Früchte (25) eines Stichprobenloses;
b) Mittel (21, 22) zum Wiegen jeder Frucht der Stichprobe;

c) Mittel (26) zur Messung der Härte dieser Früchte;

d) Mittel (35), um den Saft dieser Früchte zu entziehen;

e) Mittel (42) zur Messung des Zuckergehalts des entzogenen Safts;

f) Mittel (46, 47) zum Sammeln der Gesamtheit des entzogenen Safts;

g) Mittel (51) zum Wiegen der Gesamtheit des entzogenen Safts;

h) Mittel (43, 44a) zum Sammeln einer Teilmenge des entzogenen Safts;

i) Mittel (58, 59, 62) zur Messung des Säuregehalts der Teilmenge des Safts, der aus dem Stichprobenlos entzogen wurde,

und daß die in b), c), e), g) und i) durchgeführten Messungen in den Speicher des Computers (2) eingegeben werden, der diese Meßwerte mit den in seinen Speicher eingegebenen Parametern vergleicht und die Ergebnisse der Analyse und die organoleptischen Eigenschaften der Früchte angibt:

- das Verhältnis Gesamtgewicht der Früchte/Gesamtgewicht des entzogenen Safts,
- die mittlere Härte der Früchte,
- den mittleren Zuckergehalt,
- den mittleren Säuregehalt der Früchte.

3. Automatisierte Einheit nach Anspruch 2, die ein Gestell (1b) und Mittel (6, 7) zur Steuerung der für den Betrieb der Einheit notwendigen Energien aufweist, dadurch gekennzeichnet, daß sie eine im wesentlichen waagrechte Platte (13) enthält, die drehbar am Gestell (1b) montiert ist und regelmäßig auf dem Umfang der Platte verteilte, kreisförmige Öffnungen (13a) aufweist, wobei die Platte von einem Getriebemotor (16) sequentiell um einen Umdrehungsabschnitt angetrieben wird, der dem zwei Öffnungen (13a) trennenden Kreisbogen entspricht, wobei der Durchmesser der Löcher kleiner ist als der der Früchte (25), so daß die auf der Platte angeordneten Früchte teilweise in die Öffnungen eingeführt bleiben, während sie auf ihre Analyse warten.

4. Einheit nach einem beliebigen der Ansprüche 2 und 3, dadurch gekennzeichnet, daß die Platte (13) ringförmig und auf Rollen (14) drehbar montiert ist, die frei drehend auf dem Gestell angeordnet sind, daß die Platte der Einwirkung von Zentrierrollen (15) unterworfen ist, die an ihrem Umfangsrand anliegen, und daß ihr Antrieb in Drehbewegung von einem Reibungsrad (15a) bewirkt wird, das der Einwirkung von elastischen Mitteln (17) unterworfen und auf der Ausgangswelle des sequentiellen Getriebemotors (16) befestigt ist.

5. Einheit nach einem beliebigen der Ansprüche 3 und 4, dadurch gekennzeichnet, daß die Platte (13) einen Anwesenheitsdetektor (18) für die Öffnungen (13a) besitzt, welcher einem einziehbaren Blockierfinger (19) zugeordnet ist, der sich auf der Strecke der Öffnungen befindet, wobei der Finger einen geringeren Querschnitt aufweist als die Löcher (13a), damit er in sie eindringen und die Platte (13) sequentiell in einer genauen Position anhalten kann.

6. Einheit nach einem beliebigen der Ansprüche 2 bis 5, dadurch gekennzeichnet, daß die Mittel zum Wiegen jeder Frucht aus einem Anwesenheitsdetektor (24) für die Früchte bestehen, der einer einziehbaren Schale (22) mit einem kleineren Durchmesser als dem der Öffnungen (13a) der Platte zugeordnet ist und der sich auf der Strecke der Öffnungen befindet, wobei die Schale (22) von einer mit dem Computer (2) verbundenen Kraftmeßsonde (21) getragen wird und der Einwirkung von Mitteln (23) unterworfen ist, die sie in die Öffnungen eindringen lassen, um sequentiell und synchron mit dem Betrieb der Platte (13) eine Frucht der Stichprobe zum Zweck ihres Wiegens anzuheben.

7. Einheit nach einem beliebigen der Ansprüche 2 bis 6, dadurch gekennzeichnet, daß die Mittel zur Messung der Härte der Früchte aus Mitteln (30, 31 32) zum sequentiellen Blokkieren jeder Frucht synchron mit den anderen Organen der Einheit und aus einem mit einer mit dem Computer (2) verbundenen Kraftmeßsonde (28) gekoppelten Penetrometer (26) bestehen, dessen Dorn (27) synchron betätigt wird, um in die Frucht einzudringen.

8. Einheit nach Anspruch 7, dadurch gekennzeichnet, daß die Mittel zur Messung der Härte der Früchte aus einer ersten, ortsfesten, fest mit dem Gestell (1b) verbundenen Schale (30), die oberhalb der Platte und auf der Strecke der Öffnungen (13a) angeordnet ist, vor der nacheinander die Öffnungen der Platte (13) stehenbleiben, und einer zweiten, einziehbaren Schale (31) koaxial zur ersten Schale (30) bestehen, wobei die konkaven Seiten dieser Schalen (30, 31) einander gegenüberliegen; daß die zweite Schale (31) einen kleineren Durchmesser hat als die Öffnungen (13a) und von Teleskopmitteln (32, 33) betätigt wird, die sich unterhalb der Platte (13) befinden, um sie die Öffnungen (13a) nacheinander durchqueren und die Frucht (25) unabhängig von ihrer Dicke, in Anlage an die erste Schale (30) bringen zu lassen, die ein zentrales Loch (30a) aufweist, das der Dorn (27) des Penetrometers (26) durchdringt.

9. Einheit nach einem beliebigen der Ansprüche 2 bis 8, dadurch gekennzeichnet, daß sie im rechten Winkel zur Achse des Penetrometers (26) einen Auswerfer (34) für die Früchte aufweist, die die Härte-Meßstation (26) durchlaufen haben, wobei dieser Auswerfer (34) parallel zur Platte (13) liegt und zwischen der Platte und der ersten, ortsfesten Schale (30) des Penetrometers angeordnet ist.

10. Einheit nach einem beliebigen der Ansprüche 8 und 9, dadurch gekennzeichnet, daß die Teleskopmittel der zweiten Schale (31) aus zwei übereinanderliegenden Zylindern (32, 33) bestehen, von denen (32) einer die Funktion hat, die Schale (31) zu bewegen, um die Frucht an die erste, ortsfeste Schale (30) in Anlage zu bringen, und der andere Zylinder (33) zur Aufgabe hat, die Schale (31) kurzzeitig über der Platte (13) anzuhalten, um die Frucht im wesentlichen in das Wirkungsfeld des Auswerfers (34) zu bringen und sie von der Platte (13) zum Mittel (35) für den Saftentzug zu transportieren.

11. Einheit nach einem beliebigen der Ansprüche 7 bis 10, dadurch gekennzeichnet, daß das Ende (27a) des Dorns (27) des Penetrometers (26) eine konkave Ausbildung (27a$_1$) besitzt, die von einem zugespitzten peripheren Rand begrenzt wird, um die Haut der Frucht vor dem Eindringen des Dorns in das Fruchtfleisch zu zerschneiden.

12. Einheit nach einem beliebigen der Ansprüche 2 bis 11, dadurch gekennzeichnet, daß die Mittel für den Entzug des Safts aus den Früchten aus einer Presse (35) bestehen, die im wesentlichen waagrecht und unterhalb der Platte (13) angeordnet ist, wobei diese Presse eine seitliche Öffnung (36a) aufweist, um die von der Platte (13) ausgeworfenen Früchte (25) aufzunehmen.

13. Einheit nach Anspruch 12, dadurch gekennzeichnet, daß die Presse (35) aus einem an seinen Enden offenen Zylinderrohr (36) besteht, dessen Wand eine Öffnung (36a) für die Einführung der Früchte (25) mittels Schwerkraft aufweist, wobei in dieses Zylinderrohr ein Kolben (37) montiert ist, der an der beweglichen Stange (38a) eines Zylinders (38) mit doppelter Wirkung befestigt ist, um den Kolben (37) über die Länge des Zylinderrohrs zu bewegen, wobei das Zylinderrohr (36) an die bewegliche Stange eines Zylinders (40) mit doppelter Wirkung gekoppelt ist, dessen Körper am Gestell (1b) befestigt ist und dessen Stange die Funktion hat, das Zylinderrohr (36) gegen einen am Gestell befestigten Amboß (39) anzulegen und es von diesem zu entfernen, damit die Trockenmasse der Frucht durch Schwerkraft herunterfällt, nachdem der Saft unter dem vom Kolben (37) auf die am Amboß (39) anliegende Frucht ausgeübten Druck entzogen wurde, wobei am Amboß eine Abflußrohrleitung (41) für den Saft befestigt ist.

14. Einheit nach einem beliebigen der Ansprüche 2 bis 13, bei der der von diesem Amboß (39) kommende Saft von einer ersten Rohrleitung (41) kanalisiert wird, um zur Messung des Zuckergehalts durch ein Refraktormeter (42) zu fließen, und dann den Saft in ein sogenanntes Gesamtausbeute-Auffanggefäß (47) zu leiten, dadurch gekennzeichnet, daß sie weiter stromaufwärts vor dem Refraktometer (42) eine T-Röhre (43) besitzt, deren mit der ersten Rohrleitung (41) verbundenes Hauptrohr (43a) stromabwärts zum Refraktometer (42) geneigt ist und deren Ableitrohr (43b) sich unterhalb des Hauptrohrs befindet und an eine zweite Rohrleitung (44) angeschlossen ist, die in einem zweiten, sogenannten Teilausbeute-Auffanggefäß mündet, wobei die zweite Rohrleitung (44) ein erstes Ventil (48), das auf der Seite der T-Röhre (43) angeordnet ist, um in der geschlossenen Stellung des Ventils eine Stichprobe des Safts jeder Frucht des Loses aufzunehmen, und ein zweites Ventil (49) aufweist, das stromabwärts hinter dem ersten Ventil angeordnet ist, um in der geschlossenen Stellung dieses zweiten Ventils die Gesamtheit der Stichproben des Safts der Früchte eines Stichprobenloses aufzunehmen.

15. Einheit nach Anspruch 14, dadurch gekennzeichnet, daß die Auffanggefäße für die Gesamtausbeute (47) und die Teilausbeute (45) je auf einer Kraftmeßsonde (50, 51) sitzen, die am Gestell befestigt und mit dem Computer (2) verbunden ist.

16. Einheit nach einem beliebigen der Ansprüche 14 und 15, dadurch gekennzeichnet, daß sie eine Rohrleitung (60), die aus dem eine Base enthaltenden Tank kommt und in das zweite Auffanggefäß (45) der Teilausbeute mündet, an welcher Rohrleitung ein Ventil (59) eingesetzt ist, das mit Steuermitteln verbunden ist, um in das Gefäß (45) Dosen dieser Base zu liefern, einen Rührer (58) und eine mit dem Computer (2) verbundene pH-Wert-Meßsonde (62) besitzt.

17. Einheit nach einem beliebigen der Ansprüche 14 bis 16, dadurch gekennzeichnet, daß das Dosierventil (59) für die Base, der Rührer (58) und die Meßsonde (62) auf einem senkrecht beweglichen und von einem Zylinder (55) mit doppelter Wirkung betätigten Arm (54) angeordnet sind, um die Sonde (62) und den Rührer (58) in das Gefäß (45) für die Teilausbeute einzuführen oder sie daraus zu entfernen, um die Entfernung des Gefäßes (47) für die Gesamtausbeute und des Gefäßes (45) für die Teilausbeute zum Zweck ihrer periodischen Reinigung zu ermöglichen.

Fig. 1

Fig. 2

Fig. 2a

EP 0 572 341 B1

Fig. 3

Fig. 4

Fig. 5

18